# EUROPEAN PATENT APPLICATION

(11) **EP 2 377 811 A2**
(43) Date of publication of application: **19.10.2011**
(21) Application number: 09835305.5
(22) Date of filing: 24.12.2009
(51) Int. Cl.: B82B 3/00, C01G 49/00

(54) **METHOD FOR PREPARING ENGINEERED MG DOPED FERRITE SUPERPARAMAGNETIC NANO PARTICLE EXHIBITING AC MAGNETIC INDUCTION HEATING AT HIGH TEMPERATURE AND MG DOPED FERRITE SUPERPARAMAGNETIC NANO PARTICLES ENGINEERED BY THE METHOD**

(30) Priority: 24.12.2008 KR 20080133348
(71) Applicant: Nuri Vista Co., Ltd., Seoul 153-786 (KR)
(72) Inventor: BAE, Seong-Tae, Seoul 142-772 (KR); CHUNG, Kyung-Won, Incheon 405-846 (KR)
(74) Representative: Plougmann & Vingtoft A/S
(86) International application number: PCT/KR2009/007801
(87) International publication number: WO 2010/074539

(57) **Abstract**

The present invention relates to a method for preparing a ferrite superparamagnetic nano particle engineered by magnesium doping, and a technique for applying it to hyperthermia cancer cell treatment and the heat shock protein (HSP) self-defense mechanism.

## Description

### [Technical Field]

The present invention relates to a method of preparing processed super paramagnetic Mg-doped ferrite nanoparticles exhibiting high temperature self-heating characteristics and applications thereof. Here, since the processed super paramagnetic Mg-doped ferrite nanoparticles prepared by the method of the invention may be applied to in-vivo cancer hyperthermia agents and self-defense mechanism heat shock protein (HSP) derivatives, the processed super paramagnetic Mg-doped ferrite nanoparticles may be useful for clinical treatment such as brain tumor treatment, optic neuritis treatment, cerebral nerve disorder treatment, and the like.

### [Background Art]

Hyperthermia refers to a technique of heating cancer cells to a temperature 43~ 45°C to destroy the cancer cells. Since hyperthermia enables selective and effective destruction of cancer cells (tissues) locally or deeply within the body while minimizing clinical side effect as compared with conventional cancer treatment methods, such as chemotherapy and radiotheraphy, studies on clinical applicability of hyperthermia have rapidly increased.

Particularly, with the development of techniques for synthesizing super paramagnetic nanoparticles having a size of a few nanometers in recent years (after the year 2000), studies have been conducted on in-vivo hyperthermia for selective destruction of cancer cells (tissues) through direct injection of nanoparticles into the body.

Although in-vivo super paramagnetic nanoparticles currently attempted or used as a hyperthermia agent are Fe₃O₄ nanoparticles, which have been granted FDA approval, the Fe₃O₄ nanoparticles are susceptible to phase change into α-Fe₂O₃, 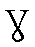 -Fe₃O₄ or Fe₃O₄ crystals depending on surrounding conditions, and heat generation characteristics and magnetic characteristics of the nanoparticles are thus changed, limiting clinical application thereof. Particularly, since the in-vivo Fe₃O₄ nanoparticles have a heat generation temperature of 15°C or less and AC frequency and magnetic field for heat generation also exceed the harmless limit to the human body, H · f = 4.85 x 10⁸ Am⁻¹s⁻¹ (H = 200 Oe or less, f = 120 kHz or less), the Fe₃O₄ nanoparticles cannot be fundamentally applied to the body.

Further, although Co, Ni or Mg-based nanoparticles such as MFe₂O₄ (M = Co, Ni or Mg) nanoparticles have also been studied, the Co, Ni or Mg-based nanoparticles cannot be applied to the body due to low heat generation temperature (Co: 25 °C, others: below 25 °C). In particular, MgFe₂O₄ provides difficulty in synthesis of nanoparticles and has low heat generation temperature due to small magnetic anisotropy.

Therefore, there is a need for nanoparticles applicable to new highly functional hyperthermia agents. In this regard, recent reports say that some Mg-based alloys or oxide nanoparticles of a few hundred nanometers to a few micrometers have high magnetization, high self-heating temperature (~120°C) and high biocompatibility (cell survival rate of 90%), thereby providing a high possibility as a hyperthermia agent. However, studies on nanoparticles having a size of 10 nm or less to be injected into the body are insufficient, and there is no method of applying the nanoparticles to the hyperthermia agent and heat shock protein derivatives for clinical treatment of brain tumors, optic neuritis, cerebral nerve disorder, and the like.

### [Disclosure]

### [Technical Problem]

One aspect of the present invention is to provide a method for synthesizing Mg-based ferrite nanoparticles, which are known to exhibit excellent biocompatibility but have difficulty in synthesis, to a size of 10 nm or less to achieve direct injection into the body, and a technique for applying magnetic characteristics of the Mg-based ferrite nanoparticles to a hyperthermia agent. The present invention suggests a possibility of applying the Mg-based nanoparticles to cancer hyperthermia agents and self-defense mechanism HSP derivatives, such that the Mg-based ferrite nanoparticles may be useful for clinical treatment of brain tumors, optic neuritis, cerebral nerve disorder, and the like.

### [Technical Solution]

In accordance with an aspect, super paramagnetic Mg-doped ferrite nanoparticles exhibiting high temperature self-heating characteristics are prepared using high temperature thermal decomposition (HTTD). For successful preparation of the super paramagnetic Mg-doped ferrite nanoparticles having various Mg amounts, reactivity of Mg ions is controlled using a reducing agent such as hexadecanediol and surfactants such as oleylamine and oleic acid. The super paramagnetic Mg-doped ferrite nanoparticles may guarantee self-heating characteristics and biocompatibility for application thereof to cancer hyperthermia and self-defense mechanism HSP derivatives.

### [Advantageous Effects]

By the method according to the invention, processed super paramagnetic Mg-doped ferrite nanoparticles are successfully prepared to have a size of 7~8 nm for direct injection into the body. The prepared super paramagnetic Mg-doped ferrite nanoparticles generate heat in a predetermined temperature range (a few °C to 100°C) and high bio-compatibility (cell survival rate: 90% or more) within the range of frequency and magnetic field applicable to the body. Through heat generation within this temperature range, the super paramagnetic Mg-doped ferrite nanoparticles may be applied to the body as hyperthermia and HSP agents.

Since the processed super paramagnetic Mg-doped ferrite nanoparticles prepared by the method according to the invention may generate heat in a predetermined range of temperatures (a few °C to 100°C) according to variation in frequency and magnetic field intensity, the super paramagnetic ferrite nanoparticles may be used in-vivo as cancer hyperthermia agents and self-defense mechanism heat shock protein (HSP) derivatives according to the kind and position of cancer cells.

The processed super paramagnetic Mg-doped ferrite nanoparticles prepared by the method according to the invention may be applied to MRI contrast media due to high magnetization value of the nanoparticles, thereby improving graphical detection techniques while enabling diagnosis and treatment to be performed at the same time in association with cancer hyperthermia.

The processed super paramagnetic Mg-doped ferrite nanoparticles prepared by the method according to the invention pave the way for protecting optic nerves and minimizing loss of eyesight caused by diabetes retina disease and glaucoma through development of effective heat shock protein technology.

### [Description of Drawings]

Fig. 1 is a flowchart of a method for preparing super paramagnetic Mg-doped ferrite nanoparticles according to the present invention;
Fig. 2 is an XRD graph depicting crystal structure of processed super paramagnetic Mg-doped ferrite nanoparticles prepared in one example of the present invention;
Fig. 3 is a transmission electron micrograph (TEM) of processed super paramagnetic Mg-doped ferrite nanoparticles prepared in another example of the present invention;
Fig. 4 is a graph depicting an analysis result of magnetic characteristics of processed super paramagnetic Mg-doped ferrite nanoparticles prepared in another example of the present invention;
Fig. 5 is a graph comparing heat generation temperature of MgFe₂O₄, FeFe₂O₄, and NiFe₂O₄ prepared by a typical method with heat generation temperature of super paramagnetic Mg-doped ferrite nanoparticles prepared in another example of the present invention;
Fig. 6 shows self-heating temperature and frequency dependency of processed super paramagnetic Mg-doped ferrite nanoparticles prepared according to the concentration of hexadecanediol in another example of the present invention;
Fig. 7 shows graphs depicting imaginary susceptibility, saturation magnetization, and power loss (total and relaxation) of super paramagnetic Mg-doped ferrite nanoparticles depending on the concentration of hexadecanediol in another example of the present invention; and
Figs. 8 and 9 show graphs depicting cell survival rates of super paramagnetic Mg-doped ferrite nanoparticles prepared in another example of the present invention, Fe₃O₄, and NiFe₂O₄, respectively.

### [Mode for Invention]

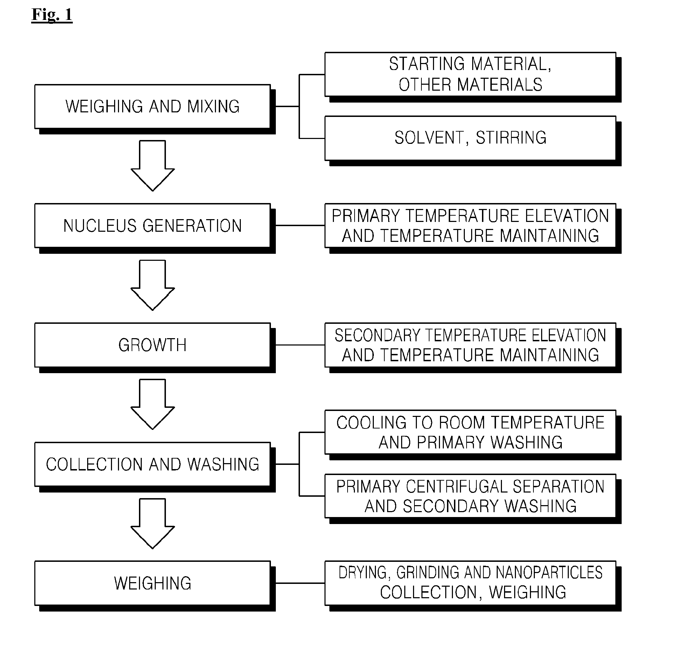

Referring to Fig. 1, a method of preparing processed super paramagnetic Mg-doped ferrite nanoparticles includes mixing raw materials, a reducing agent, benzyl ether, and surfactants, primary heat treatment to create nuclei of nanoparticles, and secondary heat treatment to grow the nanoparticles, followed by washing, centrifugal separation, drying, and grinding of the nanoparticles. Here, Fe(III) acetylacetonate and magnesium are used as the raw materials, hexadecanediol is used as the reducing agent, and oleic acid and oleylamine are used as the surfactants.

Then, the prepared nanoparticles may be coated with tetraethyl orthosilicate (TEOS), chitosan, and D-glucose dextran.

In order to ascertain applicability of cancer hyperthermia using MgFe₂O₄ nanoparticles prepared by the method according to the invention to MRI contrast media and treatment of neural diseases and optic neuritis through heat shock protein self-defense mechanism, self-heat generation characteristics, magnetic characteristics, and biocompatibility of the nanoparticles are tested.

The present invention will be described in more detail with reference to an example.

### Chemicals

1. Fe (III) acetylacetonate (2 mmol) was used as a precursor or starting material.
2. In a flask, the starting material was placed together with 1 mmol of each of target materials such as Ni, Co, and Mg according to nanoparticles to be prepared. In this example, main nanoparticles were Mg nanoparticles.
3. For reduction effect, hexadecanediol was supplied as a reducing agent to the flask while changing the amount of reducing agent from 0 to 10 mmol (0 to 100%). (The reducing agent serves as a catalyst to ionize Fe(III) acetylacetonate by separating oxygen therefrom and to help reaction between Mg ions and Fe ions to generate Mg-doped ferrite nano particles.)

### Reagents

1. 20 ml of benzyl ether was added to the mixture in the flask.
2. As surfactants, 2.4 ml of oleic acid and 3.5 ml of oleylamine were added to the mixture in the flask.

In this example, the concentration of each reducing agent was changed from 0 to 25, 50, 70 and 100% according to the presence of the surfactants to prepare processed super paramagnetic Mg-doped ferrite nanoparticles.

### Primary heat treatment

With a magnetic bar and a thermometer immersed in the mixture of the chemicals and the reagents in the flask, the temperature of the mixture was elevated while stirring the mixture.

For primary heat treatment, the temperature of the mixture was gradually elevated to 200°C over 30 minutes and maintained at 200°C for 30 minutes. This process is performed for nucleus generation.

### Secondary heat treatment

The temperature of the mixture was gradually elevated to the melting point of benzyl ether, that is, 296°C, over about 30 minutes.

Then, the mixture was maintained at the melting point of the benzyl ether for 30 minutes. This process is performed to grow the nanoparticles. Although phenyl ether can be used instead of benzyl ether, benzyl ether was used in this example in consideration of the fact that high temperature helps size distribution in preparation of the nanoparticles. After maintaining the mixture solution at the melting point for 30 minutes, the temperature of the mixture solution was lowered to room temperature.

### Primary washing and centrifugal separation

The mixture was washed for 1 hour by supplying 40 ml of ethanol to the mixture solution lowered to room temperature. After washing the mixture, the mixture was placed in a tube of a centrifugal separator, which in turn was operated at 6500 rpm for 12 minutes and 30 seconds. After operation of the centrifugal separator, it could be seen that the tube of the centrifugal separator had black deposits, that is, nanoparticles. Then, the remaining solution was removed from the tube.

### Secondary cleaning and centrifugal separation

30 ml of ethanol and 15 ml of hexane were placed in the flask and stirred using a magnetic bar to prepare a washing solution. The nanoparticles obtained in primary centrifugal separation were provided to the flask and washed using the washing solution for 40 minutes. After washing the mixture, the solution containing the nanoparticles was provided to the tube of the centrifugal separator, which in turn was operated at 6500 rpm for 12 minutes and 30 seconds. After operation of the centrifugal separator, the solution was removed from the tube of the centrifugal separator and the nanoparticles were dried at room temperature.

### Grinding

The completely dried nanoparticles were placed in a mortar and ground for about 10 minutes. The ground nanoparticles were collected and weighed for storage.

As prepared by the process shown in Fig. 1, the processed super paramagnetic Mg-doped ferrite nanoparticles may be a tertiary compound such as MgFe₂O₄, or a quaternary compound, such as MgZnFe₂O₄ or MgMnFe₂O₄.

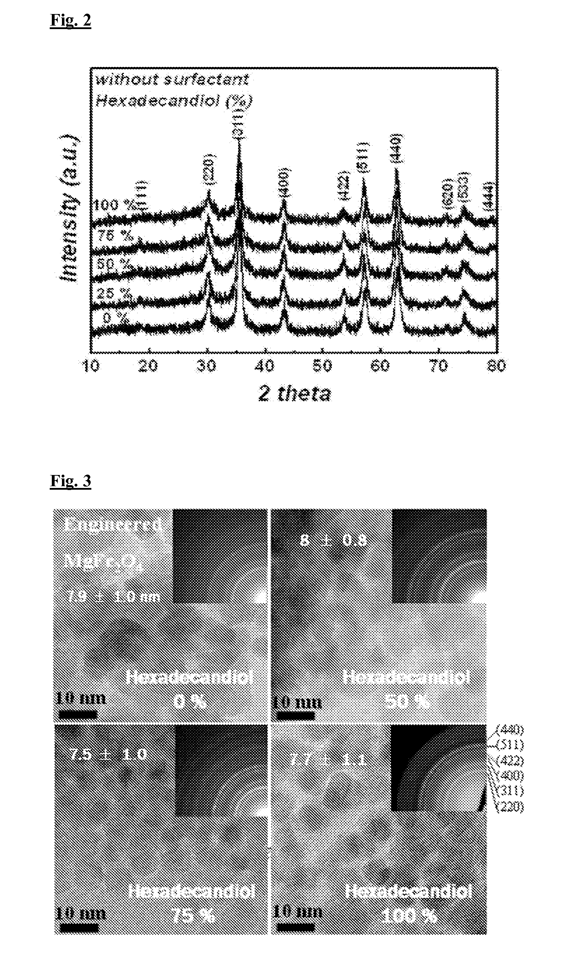

In one example, MgFe204 nanoparticles were prepared using high temperature thermal decomposition (HTTD) while changing the amounts of chemicals, that is, hexadecanediol, oleic acid and of oleylamine, from 0% to 100%. Fig. 2 and 3 show results of analyzing MgFe₂O₄ nanoparticles.

Fig. 2 is an XRD graph depicting crystal structure of the processed super paramagnetic Mg-doped ferrite nanoparticles. As can be seen from Fig. 2, the processed super paramagnetic Mg-doped ferrite nanoparticles have the same crystal structure having a single phase according to the doped amounts of Mg.

Fig. 3 is a transmission electron micrograph (TEM) of the processed super paramagnetic Mg-doped ferrite nanoparticles. As can be seen from Fig. 3, the processed super paramagnetic Mg-doped ferrite nanoparticles has a particle size of 6.5 ~ 9 nm. With these results, it can be seen that the Mg-doped ferrite nanoparticles prepared by the method according to the invention have super paramagnetic characteristics so as to be injected into the body.

Table 1 show the doped amount of Mg depending on the presence of surfactants and the concentration of hexadecandiol.

**Table 1**

| ICP/EDX | Hexadecandiol (concentration %) | Mg (atomic %) | Mg (atomic %) | Mg:Fe | |
|---|---|---|---|---|---|
| No surfactant | 0 | 1.509 | 47.606 | 0.032:1 | 0.029:1 |
| | 25 | 1.564 | 48.871 | 0.032:1 | 0.037:1 |
| | 50 | 2.044 | 43.898 | 0.047:1 | 0.035:1 |
| | 75 | 2.678 | 45.399 | 0.059:1 | 0.057:1 |
| | 100 | 2.593 | 87.860 | 0.030:1 | 0.035:1 |
| With surfactant | 0 | 0.703 | 43.186 | 0.016:1 | 0.018:1 |
| | 25 | 1.455 | 50.966 | 0.029:1 | 0.024:1 |
| | 50 | 2.175 | 80.449 | 0.027:1 | 0.022:1 |
| | 75 | 1.498 | 57.018 | 0.026:1 | 0.013:1 |
| | 100 | 1.076 | 57.054 | 0.019:1 | 0.021:1 |

From the ICP/EDX analysis results shown in Table 1, it can be seen that the doped amount of Mg is effectively controlled in the processed super paramagnetic Mg-doped ferrite nanoparticles prepared by the method according to the invention and the doped amount of Mg reaches the highest value when the nanoparticles are prepared using 75% of hexadecanediol without a surfactant.

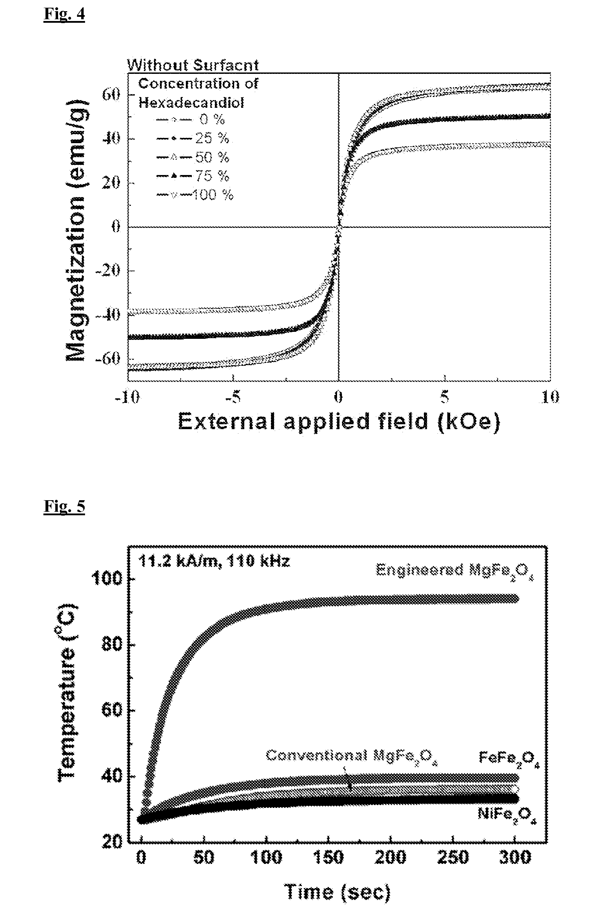

From the results of analyzing magnetic characteristics of the processed Mg-doped ferrite nanoparticles prepared by the method of the invention, it can be ascertained that the nanoparticles undergo variation in magnetization depending on the concentration of Mg and exhibit super paramagnetic characteristics without coercive force (see Fig. 4).

Heat generation characteristics of MgFe₂O₄ nanoparticles prepared according to various molar concentrations of the chemicals were measured by applying magnetic field and frequency at H · f = 4.85 x 10⁸ Am⁻¹s⁻¹ or less (H ≤12.8 kA/m, f < 120 kHz), which can be applied to the body.

When generating heat by application of a magnetic field of 140 Oe and a frequency of 110 KHz to the nanoparticles, the processed super paramagnetic Mg-doped ferrite nanoparticles containing the highest concentration of Mg, that is, 75% of hexadecanediol, exhibited a temperature of 95°C, which was higher than in other nanoparticles (Ni nanoparticles, Fe nanoparticles, Mg nanoparticles prior to grinding) by 65°C (see Fig. 5).

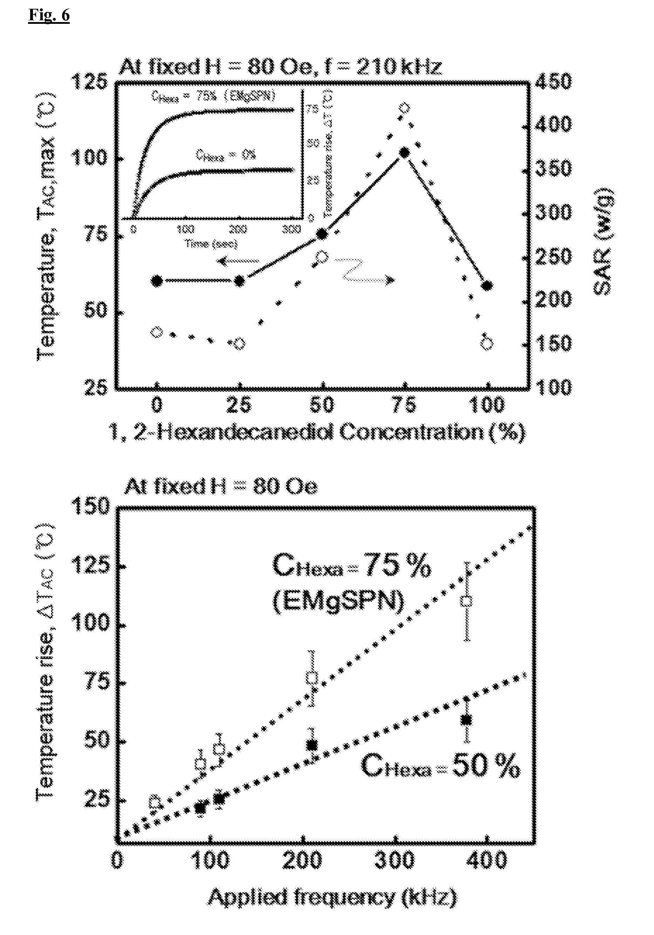

At a magnetic field intensity of 80 Oe and a frequency of 210 KHz, the processed super paramagnetic Mg-doped ferrite nanoparticles prepared by adjusting the molar concentration of the reducing agent, that is, hexadecanediol, from 0~100% without using the surfactants (oleic acid and oleylamine) exhibited various self heating temperatures from 60°C to 105°C. In this case, the nanoparticles containing the highest concentration of Mg, that is, the nanoparticles prepared using 75% of hexadecanediol, exhibited the highest self-heating temperature (Fig. 6).

Further, the processed super paramagnetic Mg-doped ferrite nanoparticles using 75% of hexadecanediol had a specific absorption rate (SAR) of 425 W/g, which was higher than any other nanoparticles containing different molar concentrations of hexadecanediol. From these results, it can be seen that the processed super paramagnetic Mg-doped ferrite nanoparticles may exhibit various self-heating temperatures through adjustment of the doped amount of Mg depending on synthesis conditions thereof. In particular, the nanoparticles prepared using 75% of hexadecanediol and containing the highest amount of Mg exhibit the highest self-heating temperature. Further, according to studies on dependency of self-heating temperature on applied frequency at a magnetic field intensity of 80 Oe with respect to the processed super paramagnetic Mg-doped ferrite nano particles, which were prepared using 75% of hexadecanediol and exhibited the highest self-heating temperature, and the processed super paramagnetic Mg-doped ferrite nanoparticles prepared using 50% of hexadecanediol, it can be ascertained that the self-heating temperature linearly increases. From this result, it can be seen that the processed super paramagnetic Mg-doped ferrite nanoparticles prepared by the method according to the present invention (in particular, nanoparticles prepared using 70% of hexadecanediol) can be applied to cancer hyperthermia and treatment of various neural diseases through self-defense mechanism heat shock protein derivatives.

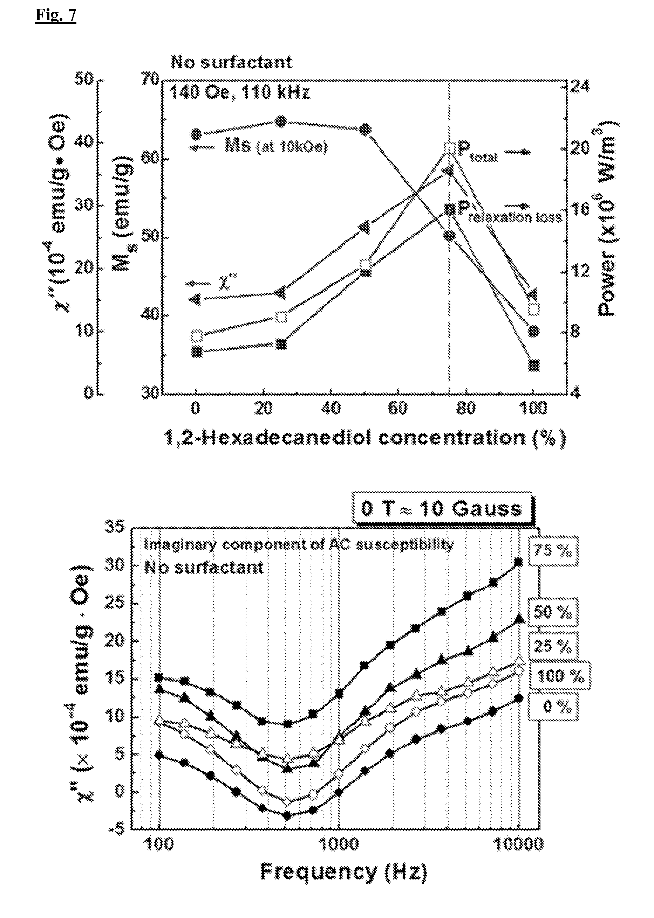

From AC-susceptibility analysis results, it is ascertained that such a remarkable increase in self-heating temperature of the processed super paramagnetic Mg-doped ferrite nanoparticles prepared using 75% of hexadecanediol is caused by a rapid increase of x" (imaginary magnetization) (see Fig. 7). Furthermore, the nanoparticles also have the highest value in terms of power loss (total and relaxation) as well as self-heating temperature and SAR.

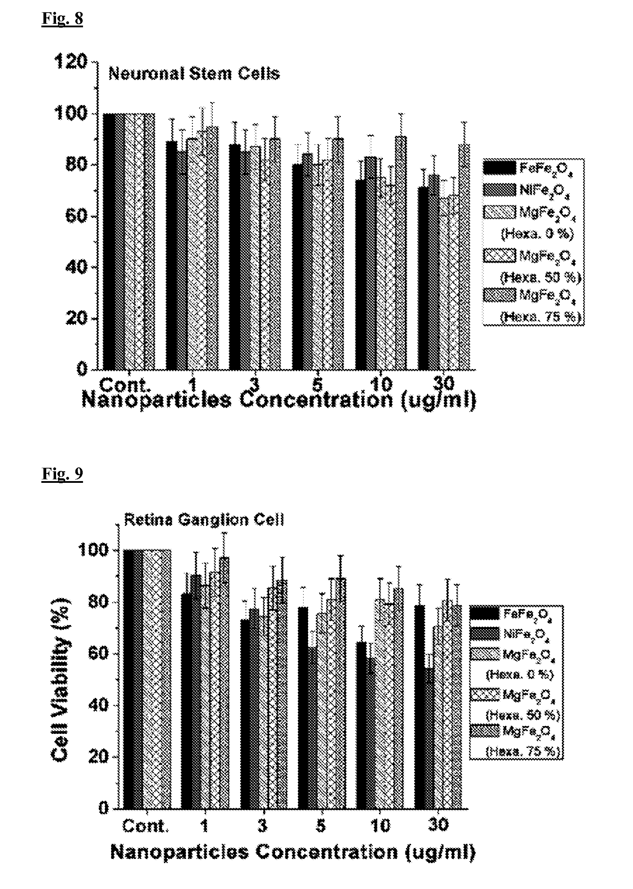

To investigate biocompatibility of the nanoparticles upon injection of the nanoparticles into the body, the cell survival rate in the processed super paramagnetic Mg-doped ferrite nanoparticles prepared by the example was examined with respect to neuronal stem cells (isolated from fetal human midbrain) and retina ganglion cells (see Figs. 8 and 9). From analysis results, the processed super paramagnetic Mg-doped ferrite nanoparticles exhibit a higher cell survival rate than not only NiFe₂O₄ nanoparticles but also FeFe₂O₄ nanoparticles, which have been granted FDA approval. Particularly, the processed super paramagnetic Mg-doped ferrite nanoparticles prepared using 70% of hexadecanediol exhibited the highest cell survival rate. From this result, it can be ascertained that the processed super paramagnetic Mg-doped ferrite prepared by the method according to the invention is suited to in-vivo application.

Since the processed super paramagnetic Mg-doped ferrite nanoparticles may be adjusted to have a high saturation magnetization of 60 emu/g in addition to the high self-heating temperature, the super paramagnetic Mg-doped ferrite nanoparticles may be applied to MRI contrast media (see Fig. 7). That is, the processed super paramagnetic Mg-doped ferrite nanoparticles prepared by the method according to the invention may be applied to cancer hyperthermia based on self-heat generation while permitting diagnosis using MRI.

As such, according to the invention, the processed super paramagnetic Mg-doped ferrite nanoparticles can completely solve problems of existing hyperthermia and HSP agents. Therefore, it is anticipated that the super paramagnetic Mg-doped ferrite nanoparticles will be used not only for destruction of cancer cells in any parts of the body but also for treatment of neural diseases such as Alzheimer's and Parkinson's disease and techniques for inducing HSP self-defense mechanism in normal cells, and will be expanded to techniques for treatment various eye diseases such as diabetes retina disease and glaucoma, thereby providing a remarkable advance in the field of medicine.

Although some embodiments have been described herein, it should be understood by those skilled in the art that these embodiments are given by way of illustration only, and that various modifications, variations, and alterations can be made without departing from the spirit and scope of the invention. Therefore, the scope of the invention should be limited only by the accompanying claims and equivalents thereof.

## Claims

1. A method of preparing processed super paramagnetic Mg-doped ferrite nanoparticles having a particle size of 10 nm or less by doping Mg into ferrite particles, comprising:
mixing a raw material comprising Fe (III) acetylacetonate, magnesium, and a solvent;
performing primary heat treatment to create nuclei of nanoparticles; and
performing secondary heat treatment to grow the nuclei to form nanoparticles, followed by washing, centrifugal separation, drying, and grinding of the nanoparticles.

2. The method of claim 1, wherein the raw material optionally further comprises hexadecanediol in a molar concentration of 10 mmol or less.

3. The method of claim 1 or 2, wherein the raw material further comprises a surfactant.

4. The method of claim 3, wherein the surfactant comprises oleic acid and oleylamine.

5. The method of claim 1, wherein the solvent comprises phenyl ether or benzyl ether.

6. The method of claim 1, wherein the primary heat treatment is performed at 200°C and the secondary heat treatment is performed at a temperature of 259 to 296°C.

7. Processed super paramagnetic Mg-doped ferrite nanoparticles prepared by the method of claim 1 and having a saturation magnetization of 40~70 emu/g.

8. The super paramagnetic Mg-doped ferrite nanoparticles of claim 7, wherein the super paramagnetic Mg-doped ferrite nanoparticles have a self-heating temperature of 10~105°C.

9. The super paramagnetic Mg-doped ferrite nanoparticles of claim 7, wherein the super paramagnetic Mg-doped ferrite nanoparticles are applied to hyperthermia.

10. The super paramagnetic Mg-doped ferrite nanoparticles of claim 9, wherein the super paramagnetic Mg-doped ferrite nanoparticles have a particle size of 10 nm or less and are applied to hyperthermia through direct injection into the body of a mammal.

11. The super paramagnetic Mg-doped ferrite nanoparticles of claim 10, wherein the super paramagnetic Mg-doped ferrite nanoparticles have a cell survival rate of 90% or more and exhibit biocompatibility.

12. The super paramagnetic Mg-doped ferrite nanoparticles of claim 10 or 11, wherein the super paramagnetic Mg-doped ferrite nanoparticles are applied to Alzheimer's or Parkinson's disease.

13. The super paramagnetic Mg-doped ferrite nanoparticles of claim 10 or 11, wherein the super paramagnetic Mg-doped ferrite nanoparticles are applied to induction of hot shock protein (HSP) derivatives in a normal cell to be applied to diabetes retina disease or glaucoma.

14. The super paramagnetic Mg-doped ferrite nanoparticles of claim 7, 10 or 11, wherein the super paramagnetic Mg-doped ferrite nanoparticles are applied to MRI contrast media.

15. The super paramagnetic Mg-doped ferrite nanoparticles of claim 14, wherein the MRI media is applied to disease diagnosis and cancer hyperthermia at the same time.

16. The method of claim 1, further comprising: coating the nanoparticles with tetraethyl orthosilicate (TEOS), chitosan and D-glucose dextran.

17. The method of claim 1, wherein the processed super paramagnetic Mg-doped ferrite nanoparticles are MgFe₂O₄, MgZnFe₂O₄, or MgMnFe₂O₄.
